# EUROPÄISCHE PATENTANMELDUNG

(11) **EP 0 880 028 A1**
(43) Veröffentlichungstag der Anmeldung: **25.11.1998**
(21) Anmeldenummer: 98108987.3
(22) Anmeldetag: 18.05.1998
(51) Int. Cl.: G01N 33/36

(54) **Prüfgerät für ein längsbewegtes Prüfgut**

(30) Priorität: 20.05.1997 CH 1164/97
(71) Anmelder: ZELLWEGER LUWA AG, 8610 Uster (CH)
(72) Erfinder: Schöni, Markus, 8603 Schwerzenbach (CH)
(74) Vertreter: Ellenberger, Maurice

(57) **Zusammenfassung**

Die Erfindung betrifft ein Prüfgerät (1) mit einer Stirnfläche (11) vor der ein Garn, Vorgarn oder Band zur Prüfung von Eigenschaften in Längsrichtung vorbeibewegt wird, mit mehreren Wirkungszonen (8, 9, 10), zur Erzeugung je einer Wechselwirkung zwischen dem Prüfgerät und dem Prüfgut. Um für das Garn, Vorgarn oder Band eine einfache, von der Zahl der Wirkungszonen unabhängige Bahn vorgeben zu können, soll das Garn, Vorgarn oder Band mindestens näherungsweise in einer Ebene quer zur Stirnfläche geführt sein.

## Beschreibung

Die Erfindung betrifft ein Prüfgerät zur Prüfung von Eigenschaffen eines, in Längsrichtung vor einer Stirnfläche vorbeibewegten, Prüfgutes, mit mehreren Wirkungszonen, zur Erzeugung je einer Wechselwirkung zwischen dem Prüfgerät und dem Prüfgut.

Bei solchen bekannten Prüfgeräten ist die Stirnfläche gerade oder aus angewinkelten Abschnitten bestehend zusammengesetzt, so dass sie beispielsweise in der Bewegungsrichtung des Prüfgutes, also beispielsweise des Garns, Vorgarns oder Bandes gerade oder insgesamt betrachtet gekrümmt sein kann. Dies ist insbesondere dann der Fall, wenn das Prüfgerät aus Modulen aufgebaut ist, welche je einen Abschnitt der Stirnfläche besetzen. Üblicherweise sind auch Führungen vorgesehen, die das Garn, Vorgarn oder Band in zwei Richtungen getrennt führen, nämlich: parallel und senkrecht zur Stirnfläche. Die Stirnfläche lässt sich in verschiedene Wirkungszonen unterteilen wie z.B. eine Prüf- oder Messzone, in der Eigenschaften des Garns, Vorgarns oder Bandes geprüft oder gemessen werden, eine Führungszone, in der Mittel zum Ausrichten und Führen des Garns, Vorgarns oder Bandes vorgesehen sind und in eine Antriebszone, in der Mittel zum Antreiben oder Bewegen des Garns, Vorgarns oder Bandes vorgesehen sind. Diese Wirkungszonen sind üblicherweise seitlich zueinander versetzt vorgesehen, so dass das Prüfgut (in Richtung auf die Stimfläche betrachtet) eine unstetige, geknickte Bahn durchläuft. Oder, mit anderen Worten, das Garn, Vorgarn oder Band wird im Bereiche der Messzone durch Führungen in eine Bahn gelenkt, die ausserhalb dieses Bereiches einen anderen Verlauf nimmt, der keine natürliche Fortsetzung der Bahn in diesem Bereiche oder in der Messzone bildet.

Die damit verbundenen Nachteile sind darin zu sehen, dass in diesem Falle Führungen immer auch Umlenkungen bewirken, die die Eigenschaffen des Garns, Vorgarns oder Bandes beeinflussen können. Beispielsweise kann die Haarigkeit eines Garns je nach dem Umschlingungswinkel des Garns auf der Führung und je nach dem Radius der Führung mehr oder weniger stark verändert werden. Ferner müssen die Wirkungszonen, die in Längsrichtung des Garns, Vorgarns oder Bandes aufeinanderfolgen sollen, seitlich versetzt vorgesehen sein, und zwar so, dass die Führungen vorausgehender und nachfolgender Wirkungszonen in ihrer Wirkung nicht beeinträchtigt werden. Dies führt entweder dazu, dass das Garn, Vorgarn oder Band für jede Wirkungszone seitlich um einen bestimmten Betrag versetzt oder ausgelenkt wird, oder dazu, dass besonders viele Führungen vorgesehen werden müssen. Zudem werden die Führungen stark belastet und belasten und verformen ihrerseits den Querschnitt des Garns, Vorgarns oder Bandes.

Die Erfindung, wie sie in den Patentansprüchen gekennzeichnet ist, löst die Aufgabe, ein Prüfgerät der genannten Art zu schaffen, das diese Nachteile nicht aufweist und für das Prüfgut eine einfache, von der Zahl der Wirkungszonen unabhängige Bahn vorgibt.

Dies wird gemäss der Erfindung dadurch erreicht, dass das Prüfgut mindestens näherungsweise in einer Ebene quer zur Stirnfläche geführt ist. Diese Ebene verläuft dabei radial (bei zweifach gekrümmter Stirnfläche) oder senkrecht zur Stirnfläche. Führungen, die für das Prüfgut eine Bahn definieren sind so angeordnet, dass eine Auslenkung des Prüfguts im Wesentlichen nur in der Ebene aber nicht in der Stirnfläche oder einer Parallelfläche dazu erfolgt. Die Führungen sind so ausgebildet, dass das Prüfgut über einen Bereich seines Umfanges geführt und vorzugsweise in der Ebene zentriert wird. Dies im Gegensatz zu bekannten Führungen, die idealerweise das Prüfgut nur längs einer einzigen Linie berühren und führen. Dies kann durch eine abgerundete, das Prüfgut teilweise umschliessende Führung oder durch mehrere Führungsflächen erreicht werden, die zueinander geneigt sind oder sich kreuzen. Die Führungsflächen sind vorzugsweise gerade oder nur wenig gekrümmt und kreuzen oder schneiden sich in der vorgenannten Ebene.

Die dadurch erreichten Vorteile sind insbesondere darin zu sehen, dass die Beeinflussung und Belastung des Prüfguts sehr gering ist. Die Anordnung einer beliebigen Anzahl Module für je eine Wirkungszone ist nun in beliebiger Reihenfolge möglich. Ebenso vereinfacht ist das automatische Einlegen des Prüfguts, denn das Prüfgut kann in einer Ebene zugeführt werden und die Führungen sind in dieser Ebene gut zugänglich. Da keine Umlenkungen vorgesehen sind, sind entsprechend nur geringere Antriebskräfte für das Prüfgut notwendig.

Im folgenden wird die Erfindung anhand eines Beispiels und mit Bezug auf die beiliegenden Figuren näher erläutert. Es zeigen
Figur 1 eine Darstellung eines erfindungsgemässen Prüfgerätes,
Figur 2 eine vereinfachte Darstellung wesentlicher Elemente des Prüfgerätes und
Figur 3 ein Einzelteil des Prüfgerätes.

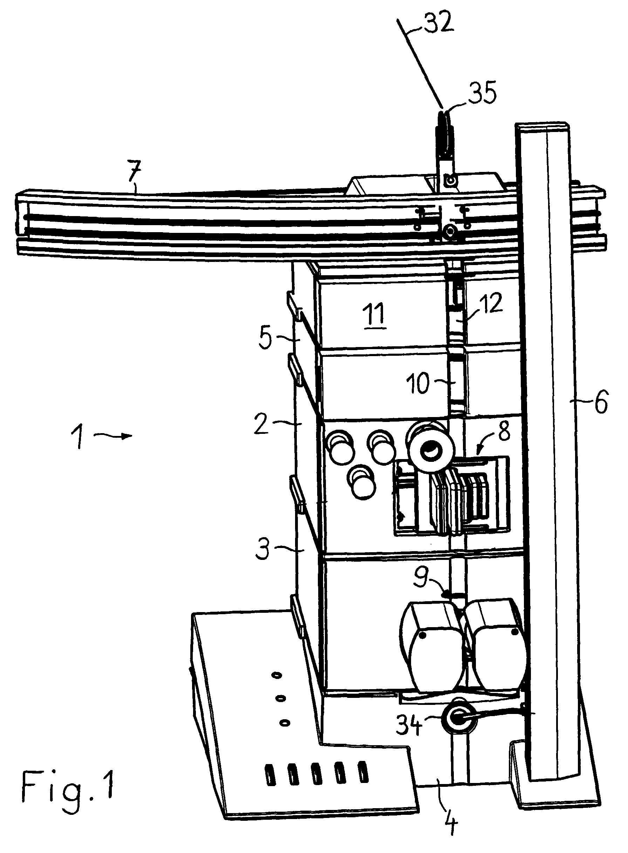

Figur 1 zeigt eine Darstellung eines erfindungsgemässen Prüfgerätes 1 mit dem Eigenschaften eines Prüfguts wie z.B. eines Garns, Vorgarns oder Bandes geprüft oder gemessen werden können. Solche Eigenschaften sind beispielsweise die Gleichmässigkeit der Masse oder des Querschnittes, die Haarigkeit, die Anwesenheit von Fremdstoffen im Prüfgut, die Zugfestigkeit, die Drehung usw. Das Prüfgerät 1 weist verschiedene Teile auf, beispielsweise verschiedene Module wie ein Prüfmodul 2, ein Antriebsmodul 3, ein Abzugsmodul 4, ein Führungsmodul 5, eine Zuführvorrichtung 6 und ein Prüfgutwechsler 7. Diese Teile und Module wirken alle in vorbestimmten Wirkungszonen in einer bestimmten Weise auf das Prüfgut ein. Man erkennt beispielsweise eine Wirkungszone 8 in der Prüf- oder Messwerte erfasst werden, eine Wirkungszone 9 in der das Prüfgut angetrieben wird und eine Wirkungszone 10 in der das Prüfgut geführt wird. Mehrere solche Wirkungszonen, in denen eine Wechselwirkung zwischen dem Prüfgut und dem Prüfgerät 1 stattfindet, sind im Bereiche, also vor oder in, einer Stirnfläche 11 und insbesondere entlang eines Kanals 12 vorgesehen, der sich über mehrere Module 2, 3, 4, 5 erstreckt.

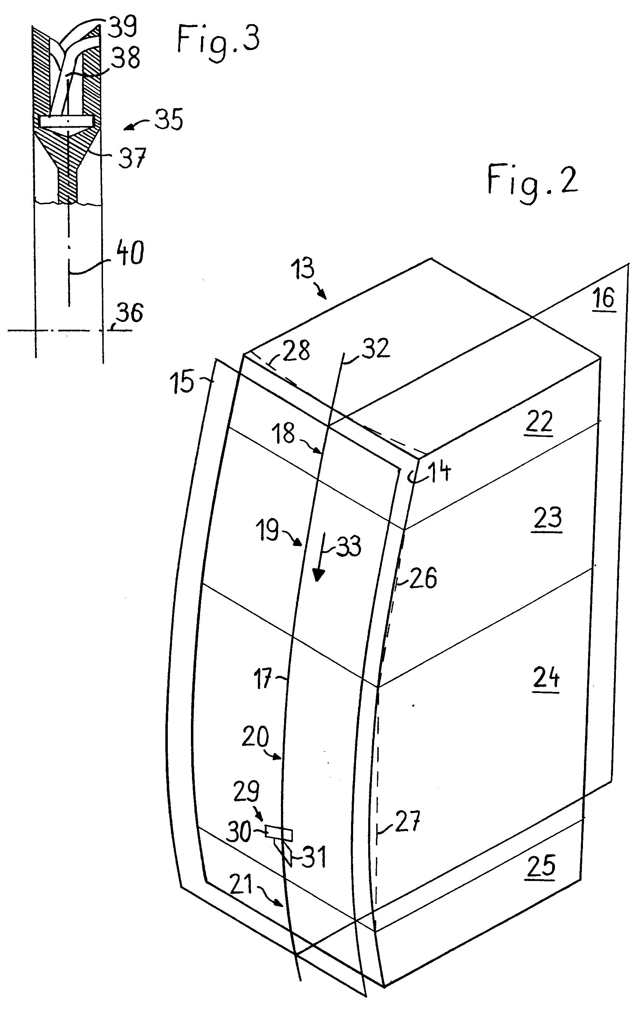

Fig. 2 zeigt ein Prüfgerät gemäss Fig. 1 nochmals in vereinfachter und schematischer Darstellung um gewisse Eigenheiten klarer erkennbar zu machen. Das Prüfgerät 13, hier ohne Prüfgutwechsler und Zuführvorrichtung gezeigt, hat eine Stirnfläche 14 und parallel dazu ist eine Parallelfläche 15 zur Stirnfläche 14 gedacht und eingezeichnet. Im Wesentlichen senkrecht zur Stirnfläche 14 und zur Parallelfläche 15 ist eine Ebene 16 gedacht angeordnet, die mit der Parallelebene 15 eine Schnittlinie 17 bildet. Diese bildet eine Bahn für das Prüfgut, insbesondere das Garn, Vorgarn oder Band, längs der es sich bewegen kann. Längs dieser Bahn oder Schnittlinie 17 sind verschiedene Wirkungszonen 18, 19, 20 und 21 vorgesehen, in denen eine Wechselwirkung zwischen dem Prüfgerät und dem Prüfgut in der Bahn 17 stattfindet. Solche Wechselwirkungen sind beispielsweise das Führen, das Messen oder Prüfen, das Antreiben oder Bewegen des Prüfguts usw. Dementsprechend sind in diesen Wirkungszonen entsprechende Mittel angeordnet. In den Wirkungszonen 18 und 21 sind dies beispielsweise Führungen für das Garn, Vorgarn oder Band. In der Wirkungszone 19 ist dies beispielsweise ein Messorgan, insbesondere ein optisch oder kapazitiv arbeitender Gleichmässigkeitssensor, ein Haarigkeitsssensor oder ein Fremdstoffsensor usw. In der Wirkungszone 20 kann dies beispielsweise eine Antriebsvorrichtung sein, die das Garn, Vorgarn oder Band 32 in Richtung eines Pfeiles 33 zieht, usw. Entsprechend den Wirkungszonen kann das Prüfgerät 1 auch in einzelne Module 22, 23, 24 und 25 unterteilt sein, die austauschbar oder vertauschbar sind. Die einzelnen Module 22 bis 25 können statt gewölbter auch gerade Stirnflächen aufweisen, wie dies für die Module 23 und 24 mit den unterbrochenen Linien 26 und 27 angedeutet ist, die zueinander geneigte Abschnitte der Stirnfläche 14 andeuten. Zusammen bilden sie eine näherungsweise gekrümmte Stirnfläche. Die Stirnfläche könnte aber auch so gekrümmt sein, wie dies durch die unterbrochene Linie 28 angezeigt ist. So könnte sich eine zweifach gekrümmte Stirnfläche ergeben. Die Ebene 16 wäre in einem solchen Falle radial oder mindestens näherungsweise senkrecht zu einer Tangentialfläche an die Stirnfläche zu legen. Es ist aber ebenso denkbar eine gerade Stirnfläche und eine gekrümmte Parallelfläche zu haben. Es ist somit nicht Bedingung, dass die Parallelfläche 15 absolut parallel zur Stirnfläche 14 verläuft, sie ist aber vor der Stirnfläche 14 angeordnet. Entlang der Bahn 17 sind mehrere Führungen vorgesehen, von denen eine hier mit 29 bezeichnet und schematisch dargestellt ist. Die Führung 29 besteht vorzugsweise aus zwei zueinander geneigten Führungsflächen 30, 31, die beispielsweise V-förmig angeordnet sind. Die Führungsflächen 30, 31 können eben oder leicht gekrümmt sein und über oder nebeneinander angeordnet sein, so dass mindestens Teile davon sich in der Ebene 16 kreuzen oder schneiden.

Fig. 3 zeigt einen Teil eines Umlenkrades 35, wie es zur Einleitung des Prüfgutes in den Kanal 12 (Fig. 1) vorgesehen ist. Die Mittelebene 40 dieses Umlenkrades liegt im wesentlichen in der Ebene 16 (Fig.2). Dieses Umlenkrad 35 weist einen Radkörper 37 auf, der um eine Achse 36 frei drehbar gelagert ist. Zur Führung des Prüfgutes sind am Umfang hintereinander geneigte Führungsstege 38, 39 angeordnet. Diese weisen einen Abstand zueinander auf, so dass das Prüfgut zwischen den Führungsstegen gerade verläuft und nicht abgestützt ist. Solche Umlenkräder sind unter dem Namen "Feed Wheels" bekannt und beispielsweise von der Firma Ascotex Limited in Burnley, England hergestellt.

Die Wirkungsweise des Prüfgerätes ist dabei wie folgt:
Ein Prüfgut 32, insbesondere ein Garn, Vorgarn oder Band, wird in an sich bekannter und deshalb hier nicht näher dargestellter Weise durch den Prüfgutwechsler 7 über das Umlenkrad 35 von einer Spule abgezogen und durch die Zuführvorrichtung 6 vor oder in den Kanal 12 gelegt. In dieser Stellung verläuft das Prüfgut idealerweise in einer Ebene 16 und damit mindestens aus einer Sicht, parallel zu der Ebene 16, vor den Modulen 2 bis 5 (Fig. 1) längs einer geraden Bahn. Sämtliche Führungen 29 längs der Bahn 17, das Umlenkrad 35 und sämtliche Wirkungszonen 18 bis 21 sind auch so ausgerichtet, dass diese Bahn 17 möglichst in einer Ebene verläuft. Für die Prüfung oder Messung wird das Prüfgut in Richtung eines Pfeiles 33 bewegt, wobei diese Richtung etwa der Längsrichtung des Garns, Vorgarns oder Bandes entspricht. Die Bewegung wird durch das Antriebsmodul 3 erzeugt. Durch diese besondere Ausrichtung der Führungen und Wirkungszonen, kann die Zuführvorrichtung 6 mit einer einfachen geradlinigen Bewegung längs des Kanals 12 mit einem Arm 34 das Prüfgut einlegen, wobei dieses automatisch richtig in die Führungen und die Wirkungszonen fällt. Durch die vorzugsweise Krümmung der Stirnfläche 11, wie sie in Fig. 2 angedeutet ist, ist dies besonders gut sichergestellt, da dann das Prüfgut sicher auf allen Führungen entlang der Bahn 17 aufliegt. Dabei ist es unerheblich, ob das Prüfgut genau in der Stirnfläche 11, 14, genau in der Parallelfläche 15, dazwischen, oder gar hinter der Stirnfläche 11, 15 verläuft. Wichtig ist, dass es im Wesentlichen in einer Ebene 16 verläuft. Vorzugsweise ist diese radial oder senkrecht zu der Stirnfläche 11, 14 angeordnet. Durch das besonders ausgebildete Umlenkrad 35 wird sichergestellt, dass das Prügut keiner Reibung ausgesetzt ist und das Prüfgut beruhigt in die Ebene 16 eintritt, so dass auch Schwingungen in Längsrichtung gedämpft sind.

Von besonderer Bedeutung ist es, dass das Prüfgut in den Wirkungszonen in der Ebene 16 verläuft und dass die Wirkungszonen entsprechend dieser Ebene ausgerichtet sind.

## Patentansprüche

1. Prüfgerät (1) zur Prüfung von Eigenschaften eines, in Längsrichtung (33) vor einer Stirnfläche (14) vorbeibewegten, Prüfgutes (32), mit mehreren Wirkungszonen (18, 19, 20, 21), zur Erzeugung je einer Wechselwirkung zwischen dem Prüfgerät und dem Prüfgut, dadurch gekennzeichnet, dass das Prüfgut in einer Bahn (17) im Wesentlichen in einer Ebene (16) quer zur Stirnfläche in den Wirkungszonen geführt ist.

2. Prüfgerät nach Anspruch 1, dadurch gekennzeichnet, dass die Ebene senkrecht zur Stirnfläche angeordnet ist.

3. Prüfgerät nach Anspruch 1, dadurch gekennzeichnet, dass mehrere Wirkungszonen (8, 9, 10, 11) entlang der Stirnfläche längs der Bahn angeordnet sind, die einem austauschbaren Modul (2, 3, 4, 5) zugeordnet sind.

4. Prüfgerät nach Anspruch 1, dadurch gekennzeichnet, dass Führungen (29) für das Prüfgut mindestens näherungsweise entlang einer Schnittlinie (17) zwischen einer Parallelfläche (15) zur Stirnfläche und der Ebene (16) angeordnet sind.

5. Prüfgerät nach Anspruch 1, dadurch gekennzeichnet, dass Wirkungszonen für Garn, Vorgarn oder Band als Prüfgut vorgesehen sind.

6. Prüfgerät nach Anspruch 1, dadurch gekennzeichnet, dass Führungsflächen (30, 31) für das Prüfgut vorgesehen sind, die sich in der Ebene (16) kreuzen.

7. Prüfgerät nach Anspruch 1, dadurch gekennzeichnet, dass Führungen (29) auf mindestens zwei Seiten des Prüfguts angeordnet sind.

8. Prüfgerät nach Anspruch 1, dadurch gekennzeichnet, dass entlang der Bahn (12) eine Umlenkrolle (35) für das Prüfgut vorgesehen ist.
